# EUROPEAN PATENT APPLICATION

(11) **EP 3 141 282 A1**
(43) Date of publication of application: **15.03.2017**
(21) Application number: 16183141.7
(22) Date of filing: 05.08.2016
(51) Int. Cl.: A61N 5/06

(54) **DEVICE FOR TREATING, PREVENTING, OR REDUCING MYOPIA, OR THE RISK THEREOF**

(30) Priority: 09.09.2015 US 201562219367 P
(71) Applicant: Rucker, Frances Joan, Boston, MA 02114 (US)
(72) Inventor: Rucker, Frances Joan, Boston, MA 02114 (US)
(74) Representative: Robinson, Simon John

(57) **Abstract**

The device provides light stimulation to photoreceptors (e.g. to cone cells and or/ to intrinsically photosensitive retinal ganglion cells) of an eye (e.g. of both eyes) of a user in a manner that is suitable for treating, preventing, or reducing myopia, or the risk thereof, in an indoor environment.

## Description

### BACKGROUND

Myopia, also known as nearsightedness, is a visual defect in which distant objects appear blurred because their images are focused in front of the retina rather than on the retina causing a retinal blur. This can occur because the eye grows longer than the focal length of the optical components. Myopia is one of the more prevalent human visual disorders, affecting up to 25% of American adults, with associated cost of correction and management having been estimated at several billion dollars per year. In some regions of the world, more than75% of people may have myopia.

It has been hypothesized that the development of the eye's refractive state is driven by many interacting influences including the eye shape, the temporal and spatial nature of visual stimuli experienced, the amount of accommodative effort and the accuracy of focus while reading.

### SUMMARY

Myopia is a condition in which the far-point of the eye is less than infinite in distance from the eye. Thus a myopic eye can see objects clearly only within a finite distance, the limit of that far distance moving closer to the eye as the level of myopia increases. Typically, the eye is too small when an animal is born and the optics of the eye focuses a distant image behind the neural retina that lies against the posterior inner wall of the eye. As the animal grows, the eye grows, and the distant image is focused on the retina. Advancing myopia is the result of the scleral ball of the eye growing too much (axial myopia), so that the eye's distance image focal point lies in front of the retina.

According to the present invention there is provided a device as set out in the accompanying claims.

In one embodiment the device for preventing and treating (including reducing) myopia comprises a display device having a front surface; and wherein a region is provided in at least one area of the front surface, or surrounding the front surface (ideally over the entire surface) of the display device, the region providing temporal visual stimulation to a user viewing the display device.

In another embodiment the presently described method for preventing and treating myopia includes providing a stand-alone display device having a front surface. The method includes providing temporal stimulation to a user viewing the display device.

In another embodiment the presently described method for preventing and treating myopia includes providing a projector or holographic display device. The method includes providing temporal stimulation to a user viewing the projected images either on a surface or superimposed on their vision in the form of a virtual reality. The temporal stimulation may be in the form of a real or artificial image.

Note that each of the different features, techniques, configurations, etc. discussed in this disclosure can be executed independently or in combination. Accordingly, the present invention can be embodied and viewed in many different ways. Also, note that this summary section herein does not specify every embodiment and/or incrementally novel aspect of the present disclosure or claimed invention. Instead, this summary only provides a preliminary discussion of different embodiments and corresponding points of novelty over conventional techniques. For additional details, elements, and/or possible perspectives (permutations) of the invention, the reader is directed to the Detailed Description section and corresponding figures of the present disclosure as further discussed below.

### BRIEF DESCRIPTION OF THE DRAWINGS

The foregoing will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
Figure 1 depicts a graph showing the change in eye length that occurs when the eye is exposed to flickering white or yellow light, with and without a blue light component, respectively, over a range of frequencies.
Figure 2 depicts a graph showing the change in refraction that occurs when the eye is exposed to flickering white or yellow light, with and without a blue light component, respectively, over a range of frequencies.
Figure 3 depicts a diagram showing a display device including a region providing retinal stimulation to a user in accordance with embodiments of the invention.
Figure 4 depicts a diagram showing a stand-alone display device providing temporal stimulation to a user in accordance with embodiments of the invention.
Figure 5 depicts a flow diagram for a method of providing temporal stimulation to a user in accordance with embodiments of the invention.

### DETAILED DESCRIPTION

The embodiments set forth below represent the necessary information to enable those skilled in the art to practice the invention and illustrate the best mode of practicing embodiments of the invention. Upon reading the following description in light of the accompanying figures, those skilled in the art will understand the concepts of the invention and recognize applications of these concepts not particularly addressed herein. It should be understood that these concepts and applications fall within the scope of the disclosure and the accompanying claims.

The preferred embodiment of the invention will now be described with reference to the accompanying drawings. The invention may, however, be embodied in many different forms and should not be construed as limited to the embodiment set forth herein; rather, this embodiment is provided so that this disclosure will be thorough and complete, and will fully convey the scope of the invention to those skilled in the art. The terminology used in the detailed description of the particular embodiment illustrated in the accompanying drawings is not intended to be limiting of the invention. In the drawings, like numbers refer to like elements.

The present invention relates generally to methods and systems for the treatment of myopia progression. It has been hypothesized that the development of the eye's refractive state is driven by many interacting influences including genetics, the eye shape, and the nature of visual stimuli experienced and accommodative lag.

For close work and reading (in young healthy eyes), the mechanism of the crystalline lens and ciliary muscles increase the crystalline lens power to focus objects that are closer than infinity. The term "accommodation" is used to indicate this action of eye's internal optics to create a more focused image on the retina. Accommodation, and the associated convergence of the eyes, involves use of internal ciliary muscles and external muscles that further increases stress to the eyeball itself. Indeed, near work has frequently been associated with myopia development. Accommodative lag is an ocular anomaly found usually in myopic subjects wherein the eyes lag behind the near focus of an object of regard such as reading print. Accommodative lag and excessive convergence of the eyes has been shown to be a risk factor in inducing myopia and accommodative lag has been found to be reduced in blue light.

Epidemiological studies have not supported the association of education and near work as a cause for myopia. Neither the subjects reading or studying habits (hours per week spent reading or using the computer) showed up as a major myopia risk factor, though the questionnaires did not inquire about whether the subjects took breaks while reading or about lighting conditions. However, the one factor that did show up in these studies was that time spent outdoors had a significant effect in reducing refractive error, and particularly in boys.

Time spent outdoors reduces myopia. Brightness of outdoor light is a factor in reducing myopia but not the only factor. Outdoor light has an even distribution of colors of light. The illuminance outdoors on a bright sunny day is around 120K compared to around 400 lux indoors and increased light levels have been shown to slow the rate of myopia development in animals. If it was just the amount of sunlight or the brightness of light, then we might expect a gradual decrease in myopia from north to the equator, but the increase is more in Asia and the latitude does not seem to be predictive.

Indoor lights, especially tungsten lights, are especially low in blue, short wavelength, light and rich in red, long wavelength light (2850 - 3100 K), while fluorescent lights have energy-rich bands distributed throughout the visible spectrum, which are dependent on the phosphors and activators present. As a result, short-wavelength-sensitive cone (S-cone) stimulation may be compromised at normal indoor illumination levels, particularly by tungsten bulbs.

Short wavelength blue light is refracted more strongly by the optics of the eye than long wavelength red light as a result of longitudinal chromatic aberration (LCA). The shorter focal length of blue light provides a stimulus for the eye to slow its growth, and produces less accommodative lag, and prevents axial elongation and myopia in some animals. If the intensity of blue light is below a certain threshold then it may make a person more prone to developing myopia.

Another difference between indoor and outdoor environments is the amount of retinal stimulation that occurs. In the indoor environment, especially with near work, the visual scene is static, while in the outdoor environment there is continual motion. In the outdoor environment the leaves on the trees are moving, the water is rippling, people and cars are moving. The movement of objects in the visual scene creates temporal retinal stimulation as the retinal image changes over time. High rates of retinal stimulation signal that the retinal image is in focus and cause a reduction in eye growth possibly through the release of the neuromodulator dopamine. The absence of any temporal stimulation causes axial elongation and myopia.

Since the epidemiological studies indicated that time spent outdoors may have beneficial effects, it was desirable to determine whether blue light, which is more prevalent in sunlight than in many indoor illuminants, affects the development of myopia. Chicks were exposed to white light that had red, green and blue components or to yellow light with only red and green components. These were called the "with" and "without" blue conditions, respectively. Temporal changes in retinal contrast were induced by increasing and decreasing the intensity of the light in a sinusoidal manner at five different flicker rates. The chicks were exposed to the with and without blue lights at slow frequencies of 0, 0.2, 1, 2 Hz and fast frequencies of 5 and 10 Hz.

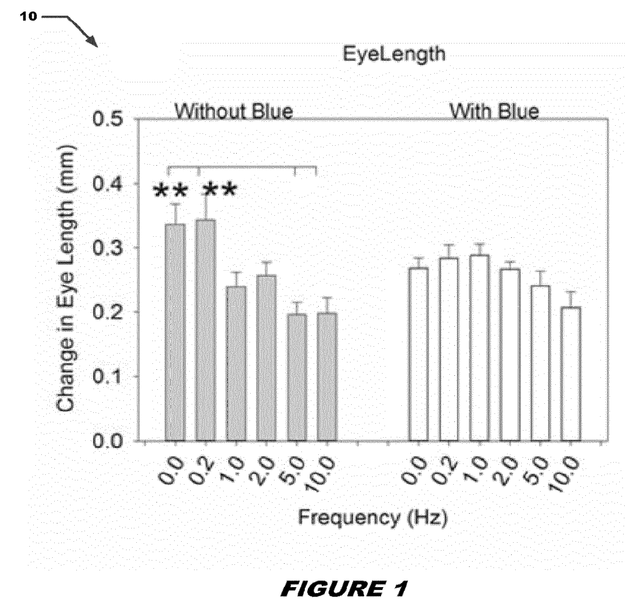

Referring to Figure 1, a graph 10 shows the difference in eye length and frequency for light with and without a blue component. Eye growth depended on whether or not blue light was present and the flicker rate. Without blue light, there was increased growth at low flicker rates and decreased growth at high flicker rates. With blue light, there was very little change in eye growth at different flicker rates. Blue light protected the eye from excessive growth at low rates of temporal stimulation.

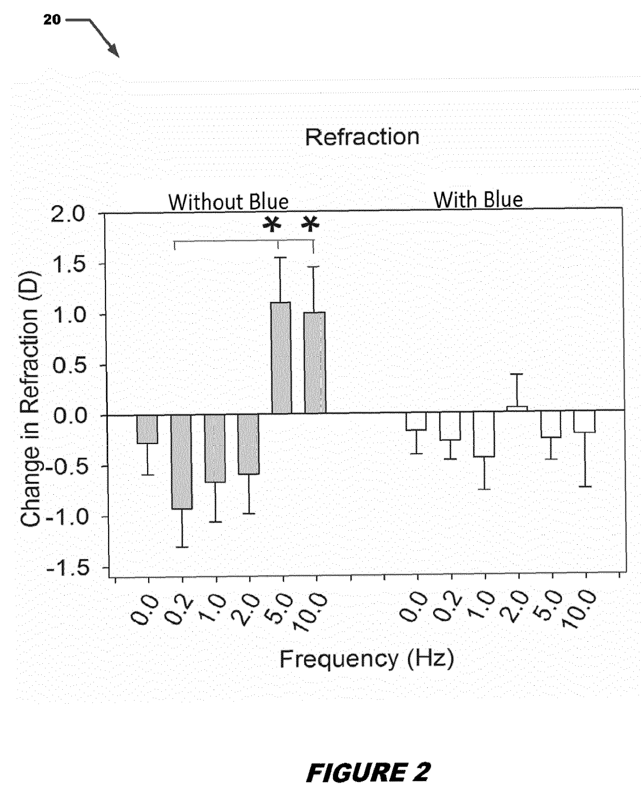

Referring to Figure 2, a graph 20 shows the difference in refraction and frequency for light with and without a blue component. Refraction changed markedly depending on whether blue light was present in the light source or not. Without blue light the eyes became hyperopic as they grew less as the flicker rate increased, while eyes became more myopic as they grew more as the flicker rate decreased. On the other hand, with blue light there was very little change in refraction at high and low flicker rates.

When the eye is exposed to a flickering light that has red, green and blue components the red, green and blue cones will detect these changes and pass signals along the neural pathways. The red and green cones will feed into a pathway that detects changes in brightness and is sensitive to rapid changes in retinal stimulation. On the other hand, the blue cone signal feeds into a pathway that detects color (among other possible destinations) and is sensitive to slow changes. The blue cone signal is known to be processed more slowly than the red and green cone signal. So at low flicker rates the eye can detect the blue stimulus, with its slower processing rate, and slow its growth, while at high flicker rates this is not possible. (In this situation it is preferred that blue light is out-of-phase with any red and green components.)

When outdoors the high rates of retinal stimulation and plentiful blue light will prevent changes in eye growth and refraction. When the light source does not contain sufficient blue light and the eye is exposed to slow changes in retinal stimulation the eye will grow more and become more myopic. This effect disappears when blue light is added to the illuminant. One suggestion is to increase the blue component of indoor lighting by using Light Emitting Diodes (LEDs) with "daylight" labeling to protect against changes in eye growth.

Referring to Figure 2, a graph 20 shows the difference in refraction and frequency for light with and without a blue component. It is desirable to stimulate the eye with high temporal frequencies when a person is indoors, particularly while looking at a computer screen, or while reading, to provide increased retinal stimulation to slow down the growth of the eye and prevent myopia.

In view of the graphs Figure 1 (graph 10) and Figure 2 (graph 20) using blue light at any frequency helps to maintain normal growth rates. Using light without blue light at higher frequencies is more effective at reducing growth and myopia than using light with a blue light component, but using light without blue light at lower frequencies causes increased eye growth and increased myopia.

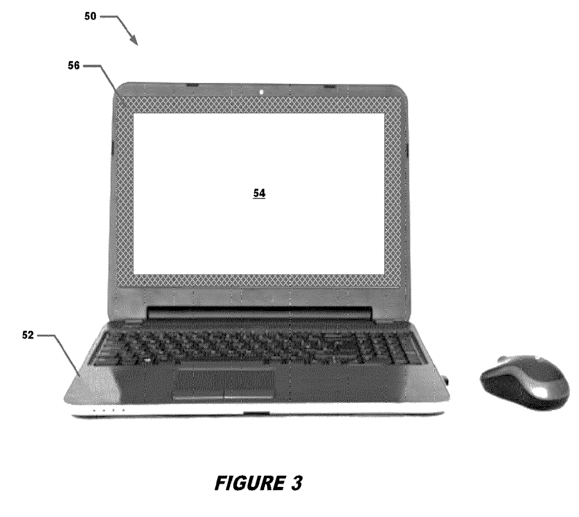

Referring now to Figure 3, a first embodiment of the present invention provides a computer 50 having a display 54 having high temporal frequencies of stimulation in the periphery 56 of the computer screen. This can be accomplished for example by having a peripheral image of a rapidly changing pattern that provides temporal stimulation. This provides the requisite temporal stimulation and can be produced with, or without, blue light, although the temporal stimulation without blue light is preferable at high temporal frequencies. Ideally, the temporal stimulation would be produced throughout the computer screen but this may not be tolerable for some people.

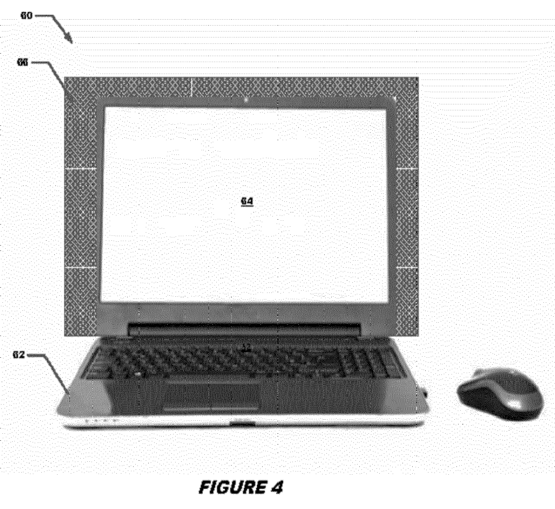

Referring to Figure 4, another embodiment is shown. Computer 62 has a display 64. An image 66 is provided behind the display positioned to extend beyond the periphery of the computer display (or reading material) and provides the temporal stimulation, with or without blue light, although the temporal stimulation without blue light is preferable at high temporal frequencies.

Alternatively, temporal stimulation could be projected on a wall behind the computer display or reading material. In other embodiments, the temporal stimulation could be provided as a projection on the lens of a pair of glasses, goggles, virtual reality type system, or the like.

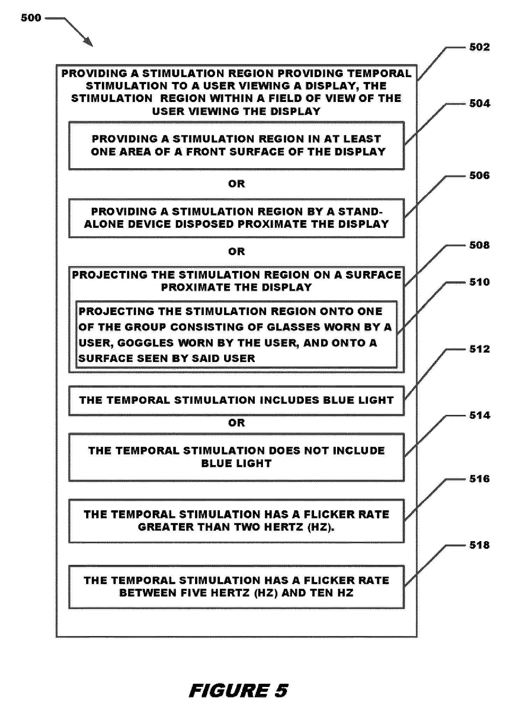

A flow chart of a particular embodiment of the presently disclosed method is depicted in Figure 5. The rectangular elements are herein denoted "processing blocks" and represent computer software instructions or groups of instructions. Alternatively, the processing blocks represent steps performed by functionally equivalent circuits such as a digital signal processor circuit or an application specific integrated circuit (ASIC). The flow diagrams do not depict the syntax of any particular programming language. Rather, the flow diagrams illustrate the functional information one of ordinary skill in the art requires to fabricate circuits or to generate computer software to perform the processing required in accordance with the present invention. It should be noted that many routine program elements, such as initialization of loops and variables and the use of temporary variables are not shown. It will be appreciated by those of ordinary skill in the art that unless otherwise indicated herein, the particular sequence of steps described is illustrative only and can be varied without departing from the spirit of the invention. Thus, unless otherwise stated the steps described below are unordered, meaning that, when possible, the steps can be performed in any convenient or desirable order.

Method 500 begins with processing block 502 which discloses providing a stimulation region providing temporal stimulation to a user viewing a display, said stimulation region within a field of view of said user viewing said display.

Processing block 504 states wherein the providing a stimulation region comprises providing a stimulation region in at least one area of a front surface of the display (ideally over the entire display). As shown in Figure 3, in one embodiment an area surrounding the outermost portions of the display screen provides temporal stimulation.

Processing block 506 recites wherein the providing a stimulation region comprises providing a stimulation region by a stand-alone device disposed proximate the display. As shown in figure 4, a display or other device is provided behind the display screen but still within the field of view of the user when the user is viewing the display screen.

Processing block 508 discloses wherein the providing a stimulation region comprises projecting the stimulation region on a surface proximate the display. As further shown in processing block 510, in certain embodiments the providing a stimulation region comprises projecting the stimulation onto one of the group consisting of glasses worn by a user and goggles worn by the user, on to a surface seen by the observer, or projecting by any other means the stimulation into the eye of the user, in effect creating a virtual reality scenario where the stimulation is superimposed on a person's vision.

Processing block 512 states wherein the temporal stimulation includes blue light. The inclusion of blue light as part of the temporal stimulation helps protect against changes in eye growth.

Processing block 514 recites wherein the temporal stimulation does not include blue light. Typical indoor light does not include much blue light, and while using illuminants without blue light at higher frequencies helps reduce growth, and the concomitant development of myopia, more than illuminants with blue light, using illuminants without blue light at lower frequencies causes increased eye growth and increased myopia.

Processing block 516 discloses wherein the temporal stimulation has a flicker rate greater than two Hertz (Hz). The flicker rate is preferably from/between 5 to 30 Hz (e.g. from/between 5 to 10 Hz, from/between 5 to 15 Hz or from/between 15 Hz to 30 Hz). Processing block 518 simply gives a non-limiting example, where the flicker rate is between 5 to 10 Hz.

Unless otherwise stated, use of the word "substantially" may be construed to include a precise relationship, condition, arrangement, orientation, and/or other characteristic, and deviations thereof as understood by one of ordinary skill in the art, to the extent that such deviations do not materially affect the disclosed methods and systems.

Throughout the entirety of the present disclosure, use of the articles "a" or "an" to modify a noun may be understood to be used for convenience and to include one, or more than one of the modified noun, unless otherwise specifically stated.

Elements, components, modules, and/or parts thereof that are described and/or otherwise portrayed through the figures to communicate with, be associated with, and/or be based on, something else, may be understood to so communicate, be associated with, and or be based on in a direct and/or indirect manner, unless otherwise stipulated herein.
Although the methods and systems have been described relative to a specific embodiment thereof, they are not so limited. Obviously many modifications and variations may become apparent in light of the above teachings. Many additional changes in the details, materials, and arrangement of parts, herein described and illustrated, may be made by those skilled in the art.

Having described preferred embodiments of the invention it will now become apparent to those of ordinary skill in the art that other embodiments incorporating these concepts may be used. Additionally, the software included as part of the invention may be embodied in a computer program product that includes a computer useable medium. For example, such a computer usable medium can include a readable memory device, such as a hard drive device, a CD-ROM, a DVD-ROM, or a computer diskette, having computer readable program code segments stored thereon. The computer readable medium can also include a communications link, either optical, wired, or wireless, having program code segments carried thereon as digital or analog signals.

It should also be noted that although much of the foregoing description discusses cone cells, the present invention may also achieve beneficial effects in treating, preventing or reducing myopia by acting upon intrinsically photosensitive retinal ganglion cells (e.g. cones and or/ intrinsically photosensitive retinal ganglion cells). Devices that reduce myopia by stimulating/acting on intrinsically photosensitive retinal ganglion cells are therefore also within the scope of the present invention.

The invention is of course not limited to only certain embodiments described above, but is limited only by the spirit and scope of the appended claims.

## Claims

1. A device that provides light stimulation to photoreceptors (e.g. to cones and/or to intrinsically photosensitive retinal ganglion cells) of an eye (e.g. of both eyes) of a user in a manner that is suitable for treating, preventing, or reducing myopia, or the risk thereof, in an indoor environment.

2. A device according to claim 1, wherein the stimulation is provided at a frequency of/at least/more than 2 Hz, at least/more than 5 Hz or at least/more than 10 Hz.

3. A device according to claim 2; wherein the stimulation is provided at a frequency of from/between 5 to 30 Hz (e.g. of from/between 5 to 10 Hz, from/between 5 to 15 Hz or from/between 15 Hz to 30 Hz).

4. A device according to any of claims 1 or 3; wherein the stimulation is in the form of one or more of the following: flickering light, an image (e.g. a fixed or changing/moving image) a pattern (e.g. a fixed or changing/moving pattern) , visual noise/visual distortions (e.g. white noise - a random pattern with a constant power spectral density, pink noise - having equal energy per octave, and so having more low-frequency components than white noise, black noise - a frequency spectrum of predominantly zero power level over all frequencies except for a few narrow bands or spikes, noisy white or black - a non-uniformity in the white or black area of the image, brown noise - random changes in an image, or any type of visual noise.)

5. A device according to any preceding claim; wherein the stimulation comprises sine wave stimulation and/or square wave stimulation.

6. A device according to any preceding claim; wherein the device
a) emits white light (i.e. light at a wavelength of 400-700 nm); or
b) emits yellow light (i.e. light at a wavelength of 500-700 nm); or
c) emits blue light (i.e. light at a wavelength of 400-500 nm); or
d) emits red light ((i.e. light at a wavelength of 600-700nm); or
e) emits green light (i.e. light at a wavelength of 500-600 nm).

7. A device according to any preceding claim; wherein the device emits light of an illuminance of 0.6 lux to 1000 lux (e.g. 0.6 to 500 lux).

8. A device according to any preceding claim; wherein the device is, or is part of, any of the following items, or is at least in the field of view of a user of any of the following items (e.g. by being integrated into, being attached to, being under, being over, being next to, being in the foreground of, or being in the background of any of the following):
a computer (e.g. a tablet, laptop or desktop computer), headwear (e.g. gaming headwear), eyewear (e.g. glasses or goggles), a phone (e.g. a smart phone), a projector, a visual display unit, a screen, a light or lamp (e.g. in LED form), a holographic display device, a music player (e.g. a digital music player), a tablet, a video player or video recorder, a home entertainment unit, an electronic blackboard or other viewable teaching aid, an e-reader, a cinema screen, a display unit for a vehicle (e.g. a dashboard or a windscreen display), a television, or any other display device (digital or otherwise).

9. A device according to any preceding claim; wherein the device comprises a screen that is used to display information or images to a user and said light stimulation is emitted from one or more regions of the device that are located so that they do not interfere with the display of said information or images.

10. A device according to claim 9; wherein the light stimulation is emitted from one or more of the following:
a) one or more peripheral regions of the device (e.g. from one or more regions surrounding a screen, from one or more edge or side regions of/to the screen);
b) from one or more defined areas within a central visual field;
c) from all or a substantial part of a display;
d) from a central part of a display .

11. A device that is suitable for treating, preventing, or reducing myopia, or the risk thereof, in an indoor environment, the device comprising:
a display having a front surface; and
a region provided along at least one side of said front service of said display, said region providing temporal stimulation to a user viewing the display.

12. A device that is suitable for treating, preventing, or reducing myopia, or the risk thereof, in an indoor environment, the device comprising a projected display providing temporal stimulation to a user viewing the display.

13. A device according to any of claims 1 to 12, wherein said stimulation is achieved with blue light or white light.

14. A device according to any of claims 1 to 12 claim, wherein said stimulation is achieved without blue light.

15. A device according to any of claims 1 to 12, wherein said stimulation is achieved with yellow light, or red or green light, or a combination thereof (e.g. with red or green light or with a combination of red or green light).
